# EUROPEAN PATENT APPLICATION

(11) **EP 1 878 406 A1**
(43) Date of publication of application: **16.01.2008**
(21) Application number: 06014341.9
(22) Date of filing: 11.07.2006
(51) Int. Cl.: A61F 2/12, A61B 17/34

(54) **System and method to implant a breast implant in a liner**

(71) Applicant: Fordyce, Cameron J., Berea OH 44017 (US); Nock, Richard, Berea OH 44017 (US)
(72) Inventor: Fordyce, Cameron J., Berea OH 44017 (US); Nock, Richard, Berea OH 44017 (US)
(74) Representative: Lorente Berges, Ana

(57) **Abstract**

The invention is a breast augmentation or reconstructive surgical system and procedure. It consists of a "system", comprised of a series of devices and instruments, used in tandem, to simplify, reduce time and undo soft-tissue trauma, ultimately giving a superior, long lasting aesthetic result for the patient. The system components are: a Tissue Retraction Ring (2), a Deployment Chamber (50), a Deployment Spring (42), a Breast Implant Liner Device (10), a Stabilization Tine(s) (95), a Pocket Expander (110) and a Breast Implant (100). The system uses a deployment chamber and spring to insert the breast implant liner device.

## Description

### Background of Invention

This invention relates to a breast augmentation or reconstructive surgical system and method.

### Background

Due to many reasons, patients undergo breast augmentation or reconstructive surgery. Each year hundreds of thousands of women elect to enhance their breasts for various personal reasons. However, post-operative complications do occur that generally elicit some form of human body reaction. Most notably, scar tissue forms naturally around the implant as a bodily defense mechanism to "wall itself off" or "protect itself" from the foreign implant. The resulting scar tissue formation may cause additional problems such as capsular contracture, hardening, rippling, rupture, etc. The result is usually dissatisfaction by the patient and physician and revision surgery may be necessary. There are standard procedures for doing this with some improvements over the years, but there exists a need to simplify, reduce time and undo soft-tissue trauma, thereby ultimately giving a superior, long lasting aesthetic result for the patient.

WO9904704 is for a breast surgery method and apparatus- It discloses a method and apparatus for subcutaneous target tissue mass through a cutaneous incision smaller than maximum transverse dimension of the tissue mass excised includes an axially elongated housing, tissue piercing means slidably resident within the housing and means connected to the elongated housing and resiliently radially expandable relative thereto for cutting said tissue mass to be excised.

W9600824US is for a filling tube and seal construction. It discloses an inflatable implant for use in human breast reconstruction includes a chamber which is defined by a flexible membrane. The implant also includes a valve and a flexible filling tube which passes through the valve and into the chamber.

EP0784987 is for a method of making in situ filler material for mammary, penile and testicular prosthesis and tissue expanders. It is an inflatable prosthesis which contains a dehydrated substance that forms a gel when mixed with an aqueous solution.

US6074421 is for a seamless breast prosthesis. It is a breast prosthesis or mammary implant comprising a single or multiple lumen elastic shell or envelope, for example of molded silicone, in which an aperture formed in the shell for removal from a mold is subsequently sealed with a silicone patch.

US5723006 is for a breast implant introducer. It is a tool and method for introducing an implant through an incision in a body utilizing a tube having a hole through which the implant is pressed. In the preferred embodiment, a two part tube has one opened end and one closed end.

US5258026 is for an endoscopic augmentation mammoplasty and instruments. It is a surgical procedure for breast augmentation is disclosed in which an incision is made inside the navel or umbilicus.

US4955906 is for a mammary prosthesis injector. The injector comprises a hollow tube having an opening at one end thereof.

EP0338701 is for a surgical tissue expander capable of insertion in a human body to induce formation of a pocket into which a mammary prosthesis may be inserted including an expander portion which is flaccid and is adapted to be inflated progressively in the body to induce formation of said pocket, a support plate portion which is sufficiently flexible that it may be bent or folded upon itself for insertion into or removal from the body through an incision in the body and to recover to a plate-like condition within the body, feed tube and releasable fastener preferably a snap fastener whereby the support portion and the expander portion are connected together until it is desired to release the expander portion from the support portion.

US6,146,418 is for a human body implant has a separate bladder made from a bio-compatible flexible polymer into which a mobile device or prosthesis is placed after the bladder has been introduced into a subcutaneous body pocket. The bladder completely forms an artificial scar by ingrowth of body tissue into pores in the bladder to form an artificial scar. In the case of a breast implant, a breast prosthesis of many different types can be used. The prosthesis is isolated from but is mobile in the bladder. Fluids can be withdrawn from the prosthesis without contaminating the body.

There is still a need to simplify, reduce time and undo soft-tissue trauma, giving a superior, long lasting aesthetic result for the patient.

### Summary of Invention

The invention is a breast augmentation or reconstructive surgical system and method. It consists of a "system", comprised of a series of devices and instruments, used in tandem, to simplify, reduce time and reduce undo soft-tissue trauma, ultimately giving a superior, long lasting aesthetic result for the patient. To reduce complications and improve the aesthetic and functional performance of breast implants, a breast implant liner is employed to promote tissue integration to reduce scar formation and to protect the body from the implant if ruptured. The shape of such implant liner will be spherical or oval in nature and will be at least 0.2 mm. to 0.8 mm in thickness and be 10% to 30% larger than the breast implant being used. This will allow the breast implant to move around freely within the liner to produce a more natural looking breast and aesthetic result. The liner will consist of two parts (upper and lower) attached together by means of glue, stitching, or other methods, or more preferably a one-piece design.

This breast implant lining device is made from a bio-compatible micro-porous material which is implanted in the breast cavity using a deployment system, in which a breast implant is subsequently inserted into the liner. It is important to note the liner must be biocompatible and have an inherently unique microstructure that allows tissue integration at the cellular level. It is also important to note that this liner will also act as a protective barrier if the breast implant should rupture, the liner will contain the implant filler material from migrating to other areas of the body. Various polymeric materials may be used so long as it is biocompatible, micro-porous, thin, soft and flexible in nature (non-palpable) and have an ability that allows tissue integration with the liner. This integration is extremely important because it greatly reduces the breast tissues from developing a foreign body reaction to the breast implant.

The unique microstructure or "porosity" of the polymeric lining device allows tissue in-growth at the cellular level, thus greatly reducing the probability of post-surgical complications, such as sear tissue formation, capsular contracture, rippling, rupture, etc. Such, microstructure or "porosity" should ideally be in the range of 20 to 80 microns in nature. Acceptable examples of polymeric materials, although not limited to, are silicone, expanded polytetraflouroethylene, polyethylene, hydrogels, surgical meshes, etc.

Other system components are: a Tissue Retraction Ring, a Deployment Chamber, a Deployment Spring, a Stabilization Tine(s), a Pocket Expander and a Breast Implant.

### Brief Description of Drawings

Without restricting the full scope of this invention, the preferred form of this invention is illustrated in the following drawings:
FIG 1 shows the Tissue Retraction Ring;
FIG 2 displays the Deployment Tube;
FIG 3 displays the Deployment Spring with handle;
FIG 4 displays the Breast Implant Liner;
FIG 5 displays the Stabilization Pocket of the Breast Implant Liner;
FIG 6 displays the Stabilization Tine;
FIG 7 displays the Breast Implant being positioned;
FIG 8 shows the liner and the retractable deployment system;

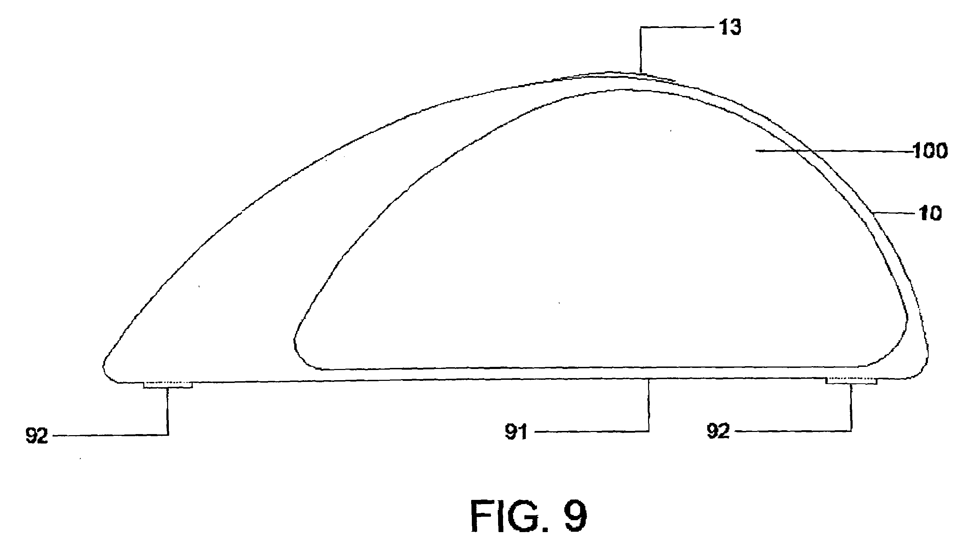

FIG 9 shows the liner with a breast implant inside; and
FIG 10 shows the pocket expanders in the liner.

### Detailed Description

The following description of a breast augmentation or reconstructive surgical procedure is demonstrative in nature and is not intended to limit the scope of the invention or its application of uses.

There are a number of significant design features and improvements incorporated within the invention.

The invention is a breast augmentation or reconstructive surgical system and surgical procedure and outcome. It consists of a method and system, comprised of a series of devices and instruments, used in tandem, to simplify, reduce time and undo soft-tissue trauma, ultimately giving a superior, long lasting aesthetic result for the patient.

To reduce complications and improve the aesthetic and functional performance of breast implants 100, a breast implant liner 10 is employed to promote tissue integration to reduce scar formation and to protect the body from the implant if ruptured. The shape of such implant liner will be spherical in nature and will be at least 0.2 mm. to 0.8 mm in thickness and be 10% to 30% larger than the breast implant being used. This will allow the breast implant to move around freely within the liner to produce a more natural looking breast and aesthetic result. The liner will consist of two parts (upper and lower) attached together by means of glue, stitching, or other methods, or more preferably a one-piece design.

The breast implant liner device 10 will be spherical or oval in shape, similar in shape and slightly larger than the breast implant 100 used, and be made from a polymeric material having a certain micro-porosity that enables tissue in-growth, and be composed of two pieces attached, or preferably, a one piece design. The device will have one or multiple dome-shaped suture tab(s) located around the outermost periphery of the implant liner device also called the liner 10, to allow easy suturing (attachment) of the liner device to the human tissues, thus stabilizing the liner to allow proper tissue integration during the healing process after implantation, and to allow for ease of breast implant insertion. The implant liner device 10 will incorporate an entrance port 94 to aid in insertion of the breast implant 100 into liner 10. The liner port 94 is closed by simply suturing, further sealing it off from the body tissues.

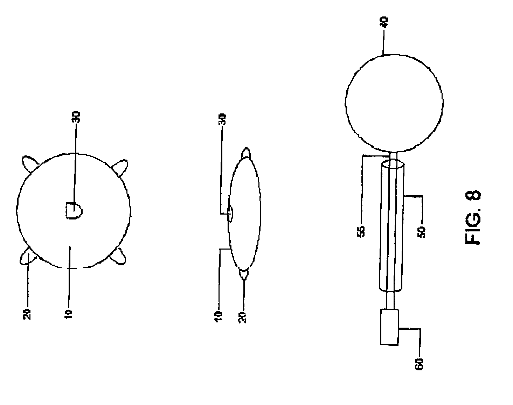

The implant liner will be inserted using a special tool called a retractable deployment system (RDS) as shown in Figure 8. The tool will be used to insert and deploy open the liner once implanted into the breast cavity to prepare the liner for the implant 100. The RDS tool 200 will be housed in a cylindrical shaped tube 50, which the liner 10 and deployment system is contained. The RDS will have a retractable spring 40 type insertion device. By pushing on the handle 60 of the RDS device, an oval-shaped wire is deployed to spread open the liner evenly into the body cavity, thus allowing for easy placement of breast implant and subsequent suturing of suture tabs or using stabilization tines placed into pockets to stabilize the liner in place. Once the liner 10 is sutured or stabilized in the desired location, the spring 40 s retracted up into the tube 50 and removed from surgical area and the implant 10 is inserted into the liner and liner sutured closed. The body incision is then sutured closed thus completing the surgery.

The system uses the following System Components:
1. Tissue Retraction Ring
2. Deployment Chamber
3. Deployment Spring
4. Breast Implant Liner Device
5. Stabilization Tine(s)
6. Pocket Expander
7. Breast Implant.

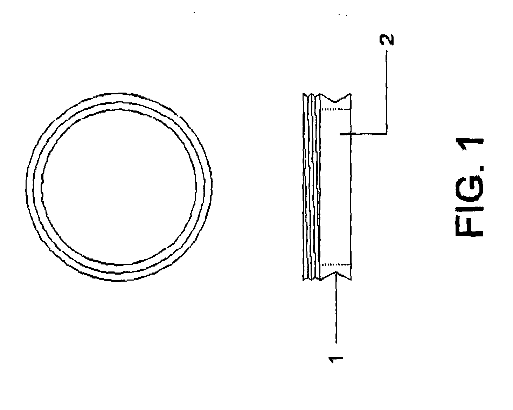

The Tissue Retraction Ring 2, as shown in Figure 1, is circular with threads 3 on the top of the ring and a tissue retraction slot 4. The Tissue Retraction Ring 2 is approximately 6 cm in diameter in the preferred embodiment and is made of s semi-rigid plastic or rubber.

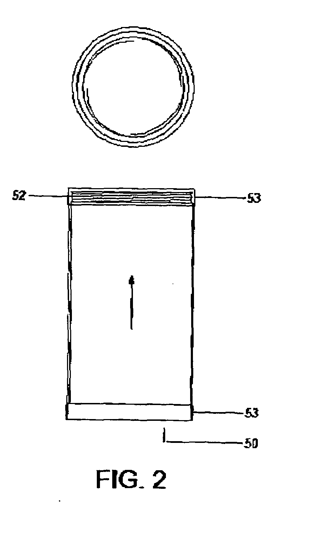

The Deployment Chamber 50, as shown in figure 2, is a circular tube with threads 52 on one end with end caps 53 and each end. It is 5 cm in diameter in the preferred embodiment. The Deployment Chamber 50 holds the Breast Implant Liner Device 10. The Deployment Chamber 50 is made of a clear semi-rigid plastic and is 6 cm in

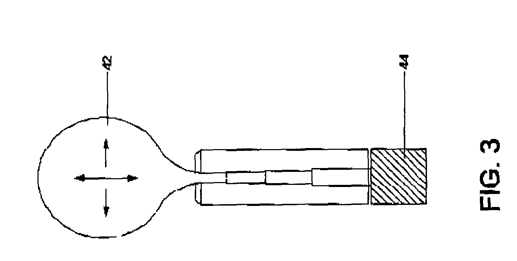

The Deployment Spring 40 has a spring load area 42 and a handle 44 and is made out of a stainless steel material in the preferred embodiment as shown in Figure 3 and Figure 8. The spring load area 42 springs out of the handle 60 carrying the Breast Implant Liner Device 10. It also unfolds the Breast Implant Liner Device 10 for placement within the patient.

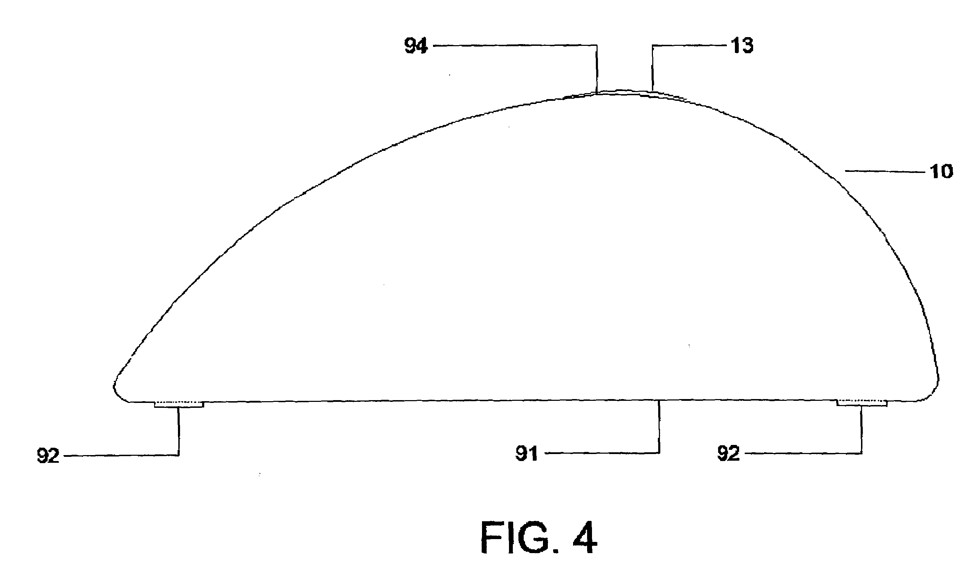

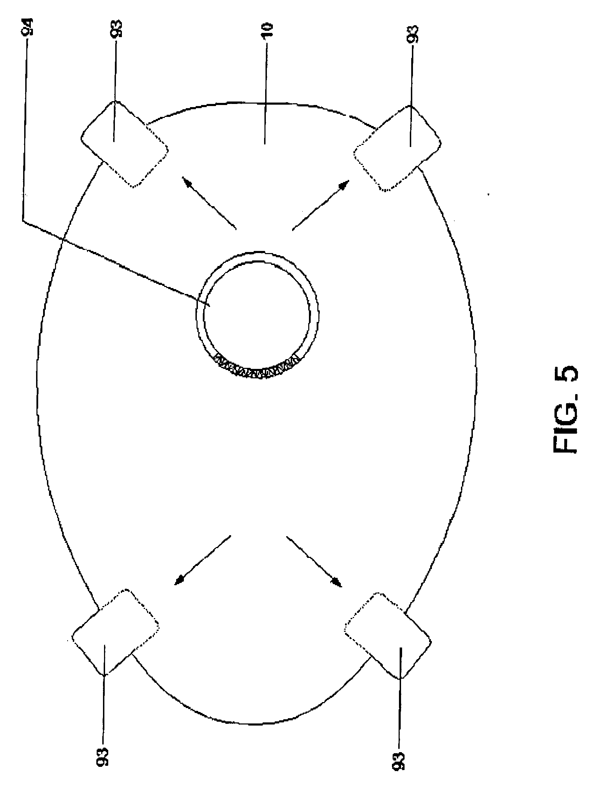

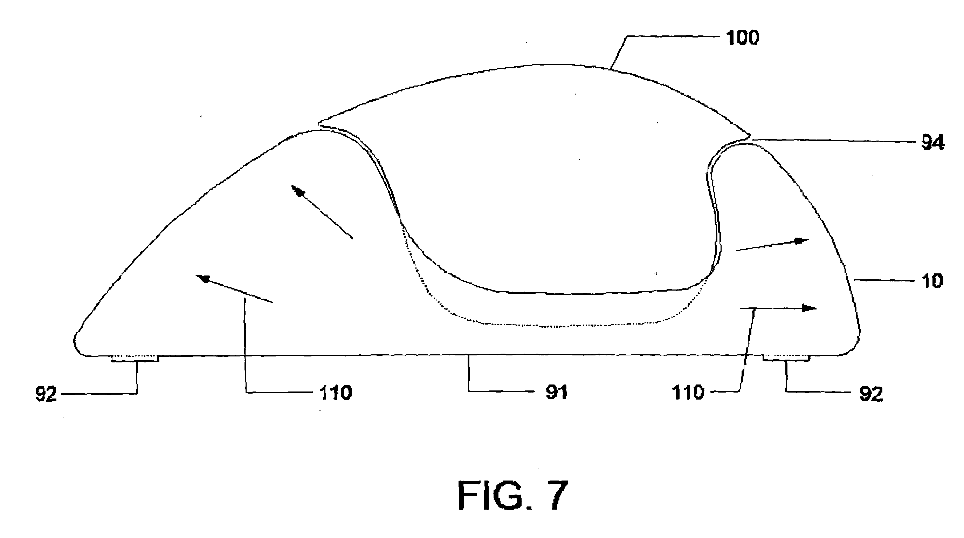

Figure 4 displays a side view of the Breast Implant Liner Device 10. The Breast Implant Liner Device 10 is a porous, bio-integrating shell made of of microporous expanded polytetrafluoroethylene (e-PTFE) which interfaces and becomes a part of and between the human body and breast implant 100. Such microstructure or "porosity" should ideally be in the range of 20 to 80 microns in nature, The base sheet 91 is the flat bottom of the Breast Implant Liner Device 10 that goes against the chest. Stabilization Pockets 92 are on the base sheet 91 and used with the Stabilizating Tine 93 to stabilize the breast implant liner device 10. The top sheet 13 is curved to provide a breast shape. There is an entrance port flap 94 in a semi-circular opening in the top sheet 13 which is unique to this invention. The breast implant is loaded into the Breast Implant Liner Device 10 through this entrance port flap 94. The Breast Implant Liner Device 10 would be made of a biological safe material. Figure 5 shows the Breast Implant Liner Device 10 from a top view and Figure 7 shows the Breast Implant Liner Device 10 from a side view. The breast implant 100 is positioned into the breast implant liner device 10 through the entrance port flap 94. The breast implant 100 can be of various sizes as desired by the patient.

The Deployment Chamber 50, as shown in figure 2, is a circular tube with threads 52 on one end with end caps 53 and each end. It is 5 cm in diameter in the preferred embodiment. The Deployment Chamber 50 holds the Breast Implant Liner Device 10. The Deployment Chamber 50 is made of a clear semi-rigid plastic or rubber and is 6 cm in

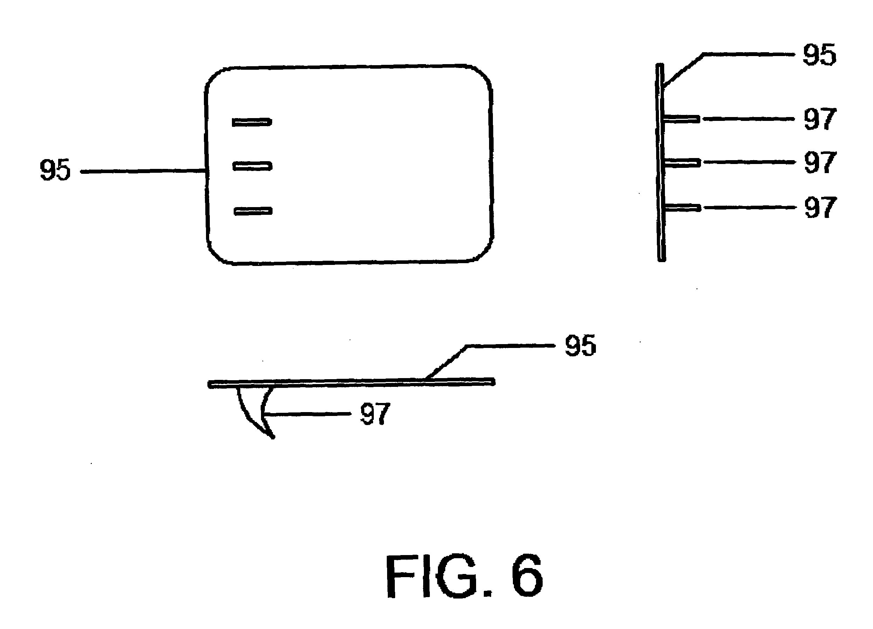

The stabilization tines 95, as shown in Figure 6 are thin and rectangular in shape with a plurality of fins that extend perpendicular for the body of the stabilization tine 95. The stabilization tines 95 fit into the stabilization pockets 92 at hold the breast implant liner device 10 in place with the fins 97 used to assist in the stabilization by extending into the chest.

The following is the steps in the procedure to install the breast implant 100 and breast implant liner 10 as covered in the current invention as shown in Figure 8.

Figure 4 displays a side view of the Breast Implant Liner Device 10. The Breast Implant Liner Device 10 is a porous, (between 5- 75 microns inner nodal distance) bio-integrating shell made of of a microporous polymer, such as expanded polytetrafluoroethylene (e-PTFE), which interfaces and becomes a part of and between the human body and breast implant. The "porosity" should ideally be in the range of 20 to 80 microns in nature. The base sheet 91 is the flat bottom of the Breast Implant Liner Device 10 that goes against the chest. Stabilization Pockets 92 are on the base sheet 91 and used with the Stabilizating Tine 93 to stabilize the breast implant liner device 10.1. *Step One:* A 4-5 cm incision port 30 is made in the nipple or below the breast fold area. This is done using generally excepted practices. The incision area is expanded using a retractor and held open. The Tissue Retraction Ring 2 is slid into the incision area, positioning the tissue (2-3 mm of skin and underlying tissue) into the "slot" on the outside of the Tissue Retraction Ring 2.

The top sheet 13 is curved to provide a breast shape. There is an entrance port flap 94 in a semi-circular opening in the top sheet 13 which is unique to this invention. The breast implant is loaded into the Breast Implant Liner Device 10 through this entrance port flap 94. The Breast Implant Liner Device 10 would be made of a biological safe material. Figure 5 shows the Breast Implant Liner Device 10 from a top view. The breast implant 100 is positioned into the breast implant liner device 10 through the entrance port flap 94. The breast implant 1.00 can be of various sizes as desired by the patient.

The stabilization tines 95, as shown in Figure 6 are biodegradable polymer thin and rectangular in shape with a plurality of fins or teeth that extend perpendicular for the body of the stabilization tine 95. The stabilization tines 95 fit into the stabilization pockets 92 at hold the breast implant liner device 10 in place with the fins used to assist in the stabilization by extending into the chest.

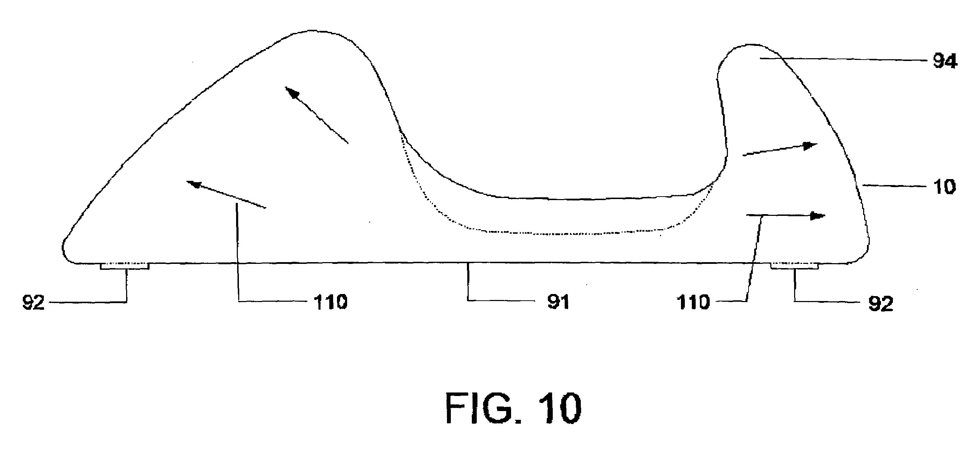

The following is the steps in the procedure to install the breast implant and breast implant liner as covered in the current invention.
1. *Step One*: A 4-5 cm incision port 30 is made in the nipple or below the breast fold area. This is done using generally excepted practices. The incision area is expanded using a retractor and held open. The Tissue Retraction Ring 2 is slid into the incision area, positioning the tissue (2-3mm of skin and underlying tissue) into the "slot" on the outside of the Tissue Retraction Ring 2. The retactor being used is removed, leaving the Tissue Retraction Ring in place, stenting open the incision. This gives a 5mm opening free of all other devices and/or instruments,
2. *Step Two:* A pocket in the breast is developed by blunt dissection, just large enough to accommodate the Breast Implant Liner Device 10 which is approximately 23 x 16cm.
3. *Step Three :* The Deployment Chamber 50 has a cap on the leading end. Unscrew the cap from the "leading end" 55 of the Deployment Chamber 50 and screw the Deployment Chamber 50 onto the Tissue Retraction Ring 2 until it "clicks" into position. The Deployment Chamber 50 will have the Breast Implant Liner Device 10 "loaded" inside, positioned to enter the breast area properly, meaning that, it is positioned so that the Breast Implant Liner Device 10 will deploy top to bottom as it is pushed into the pocket, leaving the entrance port easily retrievable.
4. *Step Four:* Insert the Deployment Spring into the Breast Implant Liner Device 10 while it is inside the Deployment Chamber. Holding onto the handle 60 of the Deployment Spring, gently "open" the Deployment Spring 40 while pushing the Breast Implant Liner Device 10 out of the Deployment Chamber 50 and maneuver the Breast Implant Liner Device 10 into position within the pocket, making sure to spread out the Breast Implant Liner Device 10 as far as possible within the pocket. Gently retract the Deployment Spring 40 back through the Tissue Retraction Ring 2 into the Deployment Chamber 50 and finally in its secure un-opened position. Unscrew the Deployment Chamber 50 from the Tissue Retraction Ring 40.
5. *Step Five:* Gently retrieve the entrance port of the Breast Implant Liner Device 10 from the pocket and pull enough material back out of the pocket as to fold over the flanged edge of the Tissue Retraction Ring. Hold edge of the entrance port and re-screw the cap of the Deployment Chamber back onto the Tissue Retraction Ring over the edges of the Breast Implant Liner Device 10. This will secure the Breast Implant Liner Device 10 open and held at the entrance port.
6. *Step Six:* Using a hemostat long and wide enough to comfortably hold the Stabilization Tine near the front edge of the Stabilization Tine 93, insert a Stabilization Tine 93 into each Stabilization, Pocket 92, located at the four "comers" 20 of the Breast Implant Liner Device 10. Great care must be employed as to not tear the Breast Implant Liner Device 10 bottom wall. A "slide and push" type technique should be used so the Stabilization Tine is all the way in the Stabilization Pocket 92. It is recommended to place the Stabilization Tine's in the 1/3/2/4 manner, meaning upper-right, lower-lett, upper-left, lower-right configuration. The Stabilization Tine's will go through the Breast Implant Liner Device and into the underlying soft-tissue and anchor the Breast Implant Liner Device 10 as tar open as possible. Push just hard enough as to stabilize but not too much as to wrinkle the bottom of the Breast Implant Liner Device 10.
7. *Step Seven:* as shown in Figure 10, Insert the unfilled Pocket Expander (Pocket Expander) 110 into the Breast Implant Liner Device 10 in the same type position as the Breast Implant Liner Device 10 (only needed with "tear-drop" design) leaving the fill tube easily accessible.
8. *Step Eight:* Lubricate the inner rim of the Tissue Retraction Ring 2 and insert the desired breast implant 100 into the Breast Implant Liner Device 10 and on top of the Pocket Expander 110. Position the breast implant so it lying approx. in the middle of the "Implant Bed" portion of the Pocket Expander 110.
9. *Step Nine:* Begin filling the Pocket Expander 110. Adjust position of the breast implant and Pocket Expander as needed. The Pocket Expander 110 should be "over-filled" and completely pressing on ALL surfaces. The breast implant 100 is used as a "pressing device" in the center portion of the Breast Implant Liner Device (generally just below the nipple or the desired "projection" location). The filled Pocket Expander 110 is left in the body for 7-10 days so as to adequately integrate tissue into the pores on the Breast Implant Liner Device 10. Sutures close the entrance port flap. Properly locate and secure the fill tube for the Pocket Expander 110 so as to be easily filled more and/or found after healing takes place.
10. *Step Ten:* Deflate Pocket Expander 110 and pull out Pocket Expander 110 from the Breast Implant Liner Device and close by suturing the incision.

If properly done, the patient will have a properly positioned Breast Implant Liner Device with a "free-floating" breast implant inside. The Breast Implant Liner Device 10 will integrate within the body, and the body will anchor the Breast Implant Liner Device 10. The Breast Implant Liner Device 10 will keep open (not allow the body to "zipper" down around the implant) the space produced by the surgery, yet because of the vacuum effect created, does not have a "balloon" effect. The Breast Implant Liner Device 10 will not allow the body to create a thick scar around the implant and will help protect the implant from the body, and the body from the implant.

Although the present invention has been described in considerable detail with reference to certain preferred versions thereof, other versions are possible. Therefore, the point and scope of the appended claims should not be limited to the description of the preferred versions contained herein.

As to a further discussion of the manner of usage and operation of the present invention, the same should be apparent from the above description. Accordingly, no further discussion relating to the manner of usage and operation will be provided.

With respect to the above description, it is to be realized that the optimum dimensional relationships for the parts of the invention, to include variations in size, materials, shape, form, function and manner of operation, assembly and use, are deemed readily apparent and obvious to one skilled in the art, and all equivalent relationships to those illustrated in the drawings and described in the specification are intended to be encompassed by the present invention.

Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the invention.

## Claims

**1.** A method to install a breast implant comprising:
cutting an incision area, forming a pocket in the breast through the incision area, having a breast implant liner in a deployment chamber, insert a deployment spring into said breast implant liner, push the deployment spring into the pocket pushing the breast implant liner out of the deployment chamber into the pocket and retrieve the deployment spring out of the pocket and installing a breast implant through an entrance port flap in the breast implant liner.

**2.** A method to install a breast implant as in Claim 1 further comprising:
positioning a Retraction Ring into the incision area expanding the incision area said retractor and held open positioning the tissue into the "slot" on the outside of the Tissue Retraction Ring and removing said retractor where said deployment chamber having a cap which connects the Deployment Chamber to said Tissue Retraction Ring.

**3.** A method to install a breast implant as in Claim 1 further comprising:
pushing the Deployment Spring through the Tissue Retraction Ring into the rocket and
retracting the Deployment Spring back through the Tissue Retraction Ring into the Deployment Chamber and unscrewing the Deployment Chamber from the Tissue Retraction Ring.

**4.** A method to install a breast implant as in Claim 1 further comprising:
having a plurality of stabilization tines inserted into stabilization pockets that are on the breast implant liner, having said Stabilization Tine go through the Breast Implant Liner and into the soft-tissue anchoring the Breast Implant Liner and using a plurality of Pocket Expanders which can be filled to expand to allow tissue to integrate into the breast implant liner and deflating the Pocket Expander and pulling the Pocket Expander out from the Breast Implant Liner and deflating the Pocket Expander and pulling the Pocket Expander out from the Breast Implant Liner.

**5.** A method to install a breast implant as in Claim 4 further comprising:
said Stabilization Tines having a plurality of teeth and being made from a semi-rigid bio-absorbable material.

**5.** A device to install a breast implant comprising:
a breast implant liner in a deployment chamber with said deployment chamber having a deployment means.

**7.** A device to install a breast implant as in Claim 6 further comprising where:
said deployment means is a handle.

**8.** A device to install a breast implant as in Claim, 7 further comprising:
said device has a deployment spring used to push said breast implant liner out of said deployment chamber.

**9.** A device to install a breast implant as in Claim 8 further comprising:
a handle on one end of said deployment chamber connected to said deployment spring.

**10.** A device to install a breast implant as in Claim 8 further comprising:
where said breast implant liner has an entrance port flap.

**11.** A device to install a breast implant as in Claim 8 further comprising:
where one end of said Deployment Chamber connects to a Tissue Retraction. Ring.

**12.** A device to install a breast implant as in Claim 11 further comprising:
where said deployment means pushes the breast implant liner through the Tissue Retraction Ring into a tissue pocket.

**13.** A device to install a breast implant as in Claim 12 further comprising:
retracting the Deployment Spring back through the into the Deployment Chamber and detaching the Deployment Chamber from the Tissue Retraction Ring.

**14.** A device to install a breast implant as in Claim 13 further comprising:
one end of said Deployment Chamber connects to a Tissue Retraction Ring by a screw threads.

**15.** A device to install a breast implant as in Claim 7 further comprising:
where said Implant Liner Device is made from a bio-compatible, flexible, micro-porous material.

**16.** A device to install a breast implant as in Claim 15 further comprising:
said material will have a porosity range between 20-80 microns and a wall thickness of between 0.3 to 0.5 millimeters.

**17.** A device to install a breast implant as in Claim 11 further comprising:
where said Tissue Retraction Ring is made from a bio-compatible semi-rigid material, having a threaded or snap fit top and a diameter of between 3 and 8 centimeters.

**18.** A device to install a breast implant as in Claim 8 further comprising:
where said Deployment Chamber and said Spring being made from semi-rigid material.

**19.** A device to install a breast implant as in Claim 5 further comprising:
having a Pocket Expander made from silicone dispersion to be inflated with saline solution and in a shape to fill said Liner.

**20.** A device to install a breast implant comprising:
a breast implant liner made from a bio-compatible, flexible, micro-porous material with an entrance port flap in a deployment chamber with said deployment chamber having a deployment means where said deployment means is a handle with a deployment spring that is used to push said breast implant liner out of said deployment chamber with the handle on one end of said deployment chamber connected to said deployment spring
where one end of said Deployment Chamber connects to a Tissue Retraction Ring which is made from made from a bio-compatible semi-rigid material, where said deployment means pushes the breast implant liner through the Tissue Retraction Ring into a tissue pocket where the Deployment Spring is retracted back through the into the Deployment Chamber and detaching the Deployment Chamber from the Tissue Retraction Ring and one end of said Deployment Chamber connects to a Tissue Retraction Ring by a screw threads.

## Amended claims

### Amended claims in accordance with Rule 137(2) EPC.

**1.** A device to install a breast implant (100) where
said deployment chamber is provided with a deployment means, consisting of a handle (44)

**2.** A device to install a breast implant as in Claim 1, further comprising:
said device has a deployment spring (40) used to push said breast implant liner (10) out of said deployment chamber (50), with said breast implant liner (10) having an entrance port flap (94).

**3.** A device to install a breast implant (100) as in Claim 2 further comprising:
a handle (44) on one end of said deployment chamber (50) connected to said deployment spring (40).

**4.** A device to install a breast (100) implant as in Claim 2 further comprising:
where one end of said Deployment Chamber (50) connects to a Tissue Retraction Ring (2), where said Tissue Retraction Ring (2) is made from a bio-compatible semi-rigid material, having a threaded or snap fit-top and a diameter of between 3 and 8 centimeters.

**5.** A device to install a breast implant (100) as in Claim 4 further comprising:
where said deployment means is able to push the breast implant liner (10) through the Tissue Retraction Ring (2) into a tissue pocket.

**6.** A device to install a breast implant (100) as in Claim 5 further comprising:
means to retract the Deployment Spring (40) back into the Deployment Chamber (50) and detaching the Deployment Chamber (50) from the Tissue Retraction Ring (2).

**7.** A device to install a breast implant (100) as in Claim 6 further comprising:
one end of said Deployment Chamber (50) connects to a Tissue Retraction Ring (2) by a screw threads (52).

**8.** A device to install a breast implant (100) as in Claim 1 further comprising:
where said Implant Liner Device (10) is made from a bio-compatible, flexible, micro-porous material.

**9.** A device to install a breast implant (100) as in Claim 8 further comprising:
said material will have a porosity range between 20-80 microns and a wall thickness of between 0.3 to 0.5 millimeters.

**10.** A device to install a breast implant (100) as in Claim 2 further comprising:
where said Deployment Chamber (50) and said Spring (40) being made from semi-rigid material.

**11.** A device to install a breast implant (100) as in Claim 1 further comprising:
having a Pocket Expander (110) made from silicone dispersion to be inflated with saline solution and in a shape to fill said Liner (10).

**12.** A device to install a breast implant (100) comprising:
a breast implant liner (10) made from a bio-compatible, flexible, micro-porous material with an entrance port flap (94) in a deployment chamber (50) with said deployment chamber having a deployment means where said deployment means is a handle (44) with a deployment spring (40) that is used to push said breast implant liner (10) out of said deployment chamber (50) with the handle (44) on one end of said deployment chamber (50) connected to said deployment spring (40) where one end of said Deployment Chamber (50) connects to a Tissue Retraction Ring (2) which is made from made from a bio-compatible semi-rigid material, where said deployment means pushes the breast implant liner (10) through the Tissue Retraction Ring (2) into a tissue pocket where the Deployment Spring (40) is retracted back into the Deployment Chamber (50) and detaching the Deployment Chamber (50) from the Tissue Retraction Ring (2) and one end of said Deployment Chamber (50) connects to a Tissue Retraction Ring (2) by a screw threads (52).
